# EUROPEAN PATENT SPECIFICATION

(11) **EP 4 346 618 B1**
(45) Date of publication and mention of the grant of the patent: **04.02.2026**
(21) Application number: 22738121.7
(22) Date of filing: 03.06.2022
(51) Int. Cl.: A61B 8/08, A61B 8/12, A61B 8/00

(54) **IMAGING CATHETER WITH SUPPORT MEMBER**
BILDGEBUNGSKATHETER MIT STÜTZELEMENT
CATHÉTER D'IMAGERIE AVEC ÉLÉMENT DE SUPPORT

(30) Priority: 04.06.2021 US 202163196850 P
(43) Date of publication of application: 10.04.2024
(73) Proprietor: Boston Scientific Scimed Inc., Maple Grove, Minnesota 55311 (US)
(72) Inventor: HANSON, Cass Alexander, St. Paul, Minnesota 55104 (US); WASDYKE, Joel M., Independence, Minnesota 55357 (US)
(74) Representative: Vossius & Partner Patentanwälte Rechtsanwälte mbB
(86) International application number: PCT/US2022/032213
(87) International publication number: WO 2022/256686

(56) References cited:
- WO-A1-95/23007
- US-A1- 2012 101 561
- US-A1- 2016 058 413
- US-A1- 2018 279 994

## Description

### Technical Field

The present disclosure pertains to medical devices, and methods for manufacturing medical devices. More particularly, the present disclosure pertains to imaging catheters.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices. Example medical imaging catheters which contain supporting members in the distal tip region are disclosed in patent documents US2016/058413A1 and WO95/23007A1.

### Brief Summary

This disclosure provides design, material, manufacturing method, and use alternatives for medical devices. An intravascular imaging catheter, as defined in claim 1, is disclosed. The intravascular imaging catheter comprises: an elongate sheath having a distal tip and a body region wherein the distal tip region includes a first port, a second port, and a guidewire lumen extending between the first port and the second port; a core wire disposed within the elongate sheath along the distal tip region and extending proximally along at least a portion of the body region; and an imaging core disposed within the elongate sheath, wherein the core wire is configured to be radially and/or circumferentially aligned with a guide wire extending along an exterior of the elongate sheath during use of the intravascular imaging catheter.

In an embodiment, the core wire has a tapered distal end.

In a furter embodiment, the core wire extends to a distal end of the elongate sheath.

In a further embodiment, the core wire extends proximally from the distal tip region of the elongate sheath.

In a further embodiment, the core wire is aligned with the guidewire lumen.

In a further embodiment, the core wire is radially aligned with the guidewire lumen.

In a further embodiment, the core wire is circumferentially aligned with the guidewire lumen.

In a further embodiment, the core wire is disposed within a wall of the elongate shaft.

In a further embodiment, the core wire is configured to be aligned with a guidewire extending through the guidewire lumen during use of the intravascular imaging catheter.

In a further embodiment, the imaging core includes an ultrasound transducer.

In a further embodiment, the imaging core is slidably disposed within the elongate sheath.

An intravascular imaging catheter, as defined in claim 12, is disclosed. The intravascular imaging catheter comprises: a sheath having a proximal end region, a body region, and a distal tip region; wherein the distal tip region includes a first port, a second port, and a guidewire lumen extending between the first port and the second port; a support member disposed within a wall of the sheath along the distal tip region; wherein the support member is aligned with the guidewire lumen; and an imaging core disposed within the sheath, the imaging core including an ultrasound transducer, wherein the support member extends along the body region, and wherein the support member is configured to be radially and/or circumferentially aligned with a guide wire extending along an exterior of the sheath during use of the intravascular imaging catheter

In one embodiment, the support member has a tapered distal end.

In a further embodiment, the support member extends along the proximal end region.

In a further embodiment, the support member is radially aligned with the guidewire lumen.

In a further embodiment, the support member is circumferentially aligned with the guidewire lumen.

In a further embodiment, the support member is configured to be aligned with a guidewire extending through the guidewire lumen during use of the intravascular imaging catheter.

In a further embodiment, the imaging core is slidably disposed within the sheath.

Further, a method for manufacturing an intravascular imaging catheter, as defined in claim 14, is disclosed.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a side view of an example medical device.
FIG. 2 is a side view of a portion of an example medical device.
FIG. 3 is a side view of a portion of an example medical device.
FIG. 4 is an example two-dimensional image of a portion of a blood vessel.
FIG. 5 is a side view of an example medical device.
FIG. 6 is a side view of an example medical device.
FIG. 7 is a cross-sectional view taken through line 7-7 in FIG. 6.
FIG. 8 is an example two-dimensional image of a portion of a blood vessel.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention which is defined by the appended claims.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

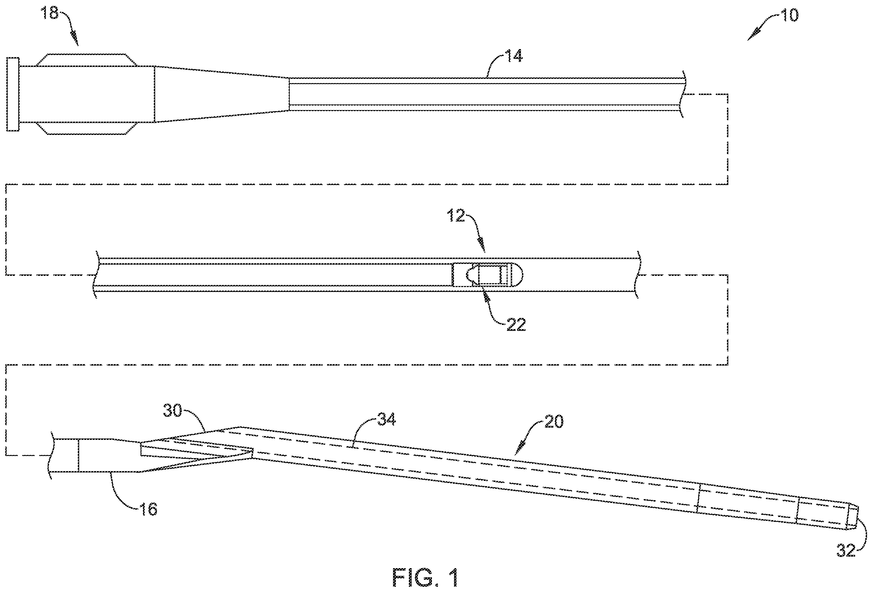

FIG. 1 is a side view of an example medical device 10. In at least some instances, the medical device 10 takes the form of an imaging medical device. For example, the medical device 10 may be an imaging device or catheter such as an intravascular ultrasound (IVUS) catheter/device that may be used to image a blood vessel. In other instances, the medical device 10 may be an imaging device that utilizes a different imaging modality such as optical coherence tomography (OCT) imaging. The structure/form of the medical device 10 can vary. In some instances, the medical device 10 may include an elongate shaft or sheath 12 having a proximal end region 14 and a body or distal end region 16. A hub 18 may be coupled to or otherwise disposed adjacent to the proximal end region 14. A distal tip region 20 may be coupled to or otherwise disposed adjacent to the distal end region 16. The distal tip region 20 may include a guidewire lumen, an atraumatic distal end, one or more radiopaque markers, and/or other features, for example as disclosed herein. For example, the distal tip region 20 may include a first port or opening 30, a second port or opening 32, and a lumen 34 (e.g., a guidewire lumen 34) extending therebetween. An imaging assembly or imaging core 22 may be disposed within the shaft 12. In general, the imaging assembly 22 may be used to capture/generate images of a blood vessel. In some instances, the medical device may include devices and/or features similar to those disclosed in U.S. Patent Application Pub. No. US 2012/0059241 and U.S. Patent Application Pub. No. US 2017/0164925. In at least some instances, the medical device 10 may resemble and/or include features that resemble the OPTICROSS^{™} Imaging Catheter, commercially available from BOSTON SCIENTIFIC, Marlborough, MA. Other imaging assemblies may also be used such as an OCT imaging assembly.

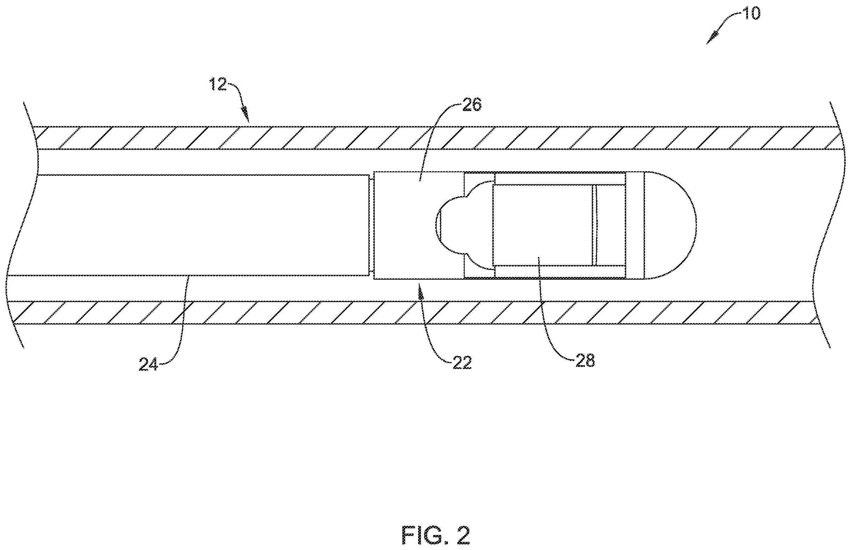

The imaging assembly 22 may include a drive cable or shaft 24, a housing 26, and an imaging member or transducer 28 coupled to the drive cable 24 and/or housing 26 as shown in FIG. 2. In at least some instances, the transducer 28 includes an ultrasound transducer. Other transducers are also contemplated. The transducer 28 may be rotatable and/or axially translatable relative to the shaft 12. For example, the drive cable 24 may be rotated and/or translated in order to rotate and/or translate the transducer 28 (and the housing 26).

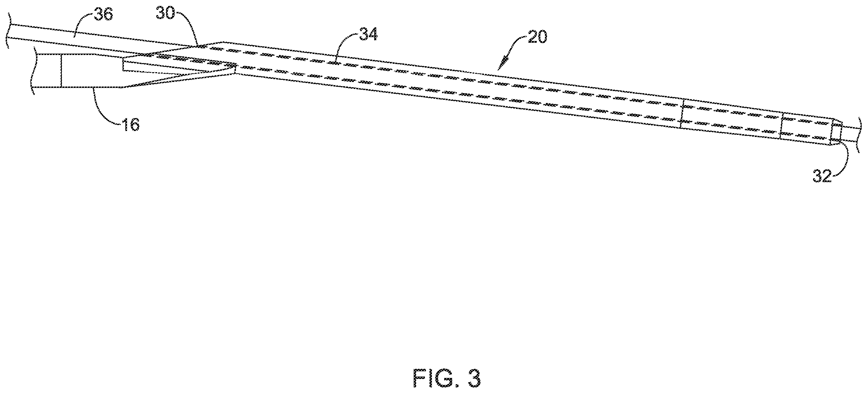

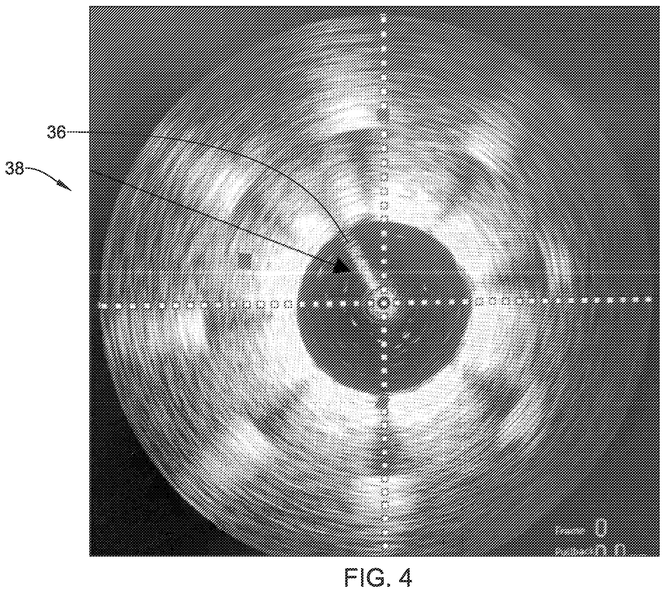

Advancing the medical device 10 through a blood vessel may include advancing the medical device 10 over a guidewire. For example, FIG. 3 illustrates a guidewire 36 passing through the lumen 34. During use, it can be appreciated that the guidewire 36 passing through the lumen 34 may be visible on a two-dimensional IVUS image 38 (e.g., an IVUS image generated by the imaging assembly 22 of the medical device 10) as depicted in FIG. 4.

In some instances, it may be desirable to increase/enhance the pushability of the medical device 10, increase/enhance the kink resistance of the medical device 10, increase/enhance the robustness of the medical device 10, and/or combinations thereof. In addition, or in the alternative, it may be desirable to increase the compatibility of the medical device 10 so that it can be used with additional guidewires (e.g., additional guidewire sizes such as 0.035 inch diameter guidewires, 0.018 inch diameter guidewires, 0.014 inch diameter guidewires, etc.). For example, the medical device 10 may be designed to be used with 0.018 inch diameter guidewires. In instances where the distal tip region 20 is adapted to be compatible with smaller (e.g. 0.014 inch diameter) guidewires, it may be desirable to provide additional structural support in order to increase/enhance pushability, increase/enhance kink resistance, increase/enhance robustness, and/or the like. Disclosed herein are medical devices that include these and other features.

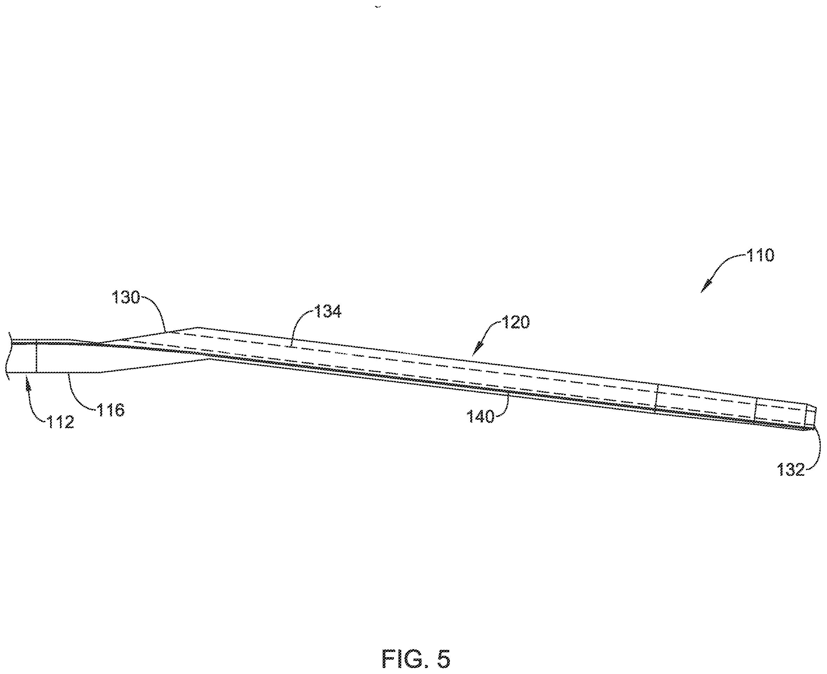

FIG. 5 illustrates a portion of another example medical device 110 that may be similar in form and function to other medical devices disclosed herein. The medical device 110 includes an elongate shaft or sheath 112 having a distal end region 116 and a distal tip region 120. An imaging assembly (not shown, but may be similar in form and function to the imaging assembly 22 disclosed herein) is disposed within the shaft 112. The distal tip region 120 includes a first port or opening 130, a second port or opening 132, and a lumen 134 (i.e. a guidewire lumen 134) extending therebetween. In at least some instances, the distal tip region 120 is adapted to be compatible with relatively small guidewires such as a 0.014 inch diameter guidewire. Other sizes/configurations are contemplated.

A support member 140 is disposed along at least a portion of the shaft 112. The support member 140 may take the form of a wire or shaft (e.g., a core wire). For example, the support member 140 may be a round wire, a ribbon-shaped wire, and/or the like. The support member 140 may have a solid cross-section along its length. Alternatively, the support member 140 may be tubular or partially tubular. The support member 140 may be formed of a suitable material such as one or more metals, polymers, and/or the like such as those disclosed herein. For example, the support member 140 may be formed from stainless steel. In general, the support member 140 may provide additional structural support to the medical device, which may increase/enhance pushability, increase/enhance kink resistance, increase/enhance robustness, and/or the like. This may be desirable regardless of what size guidewire the medical device 110 is compatible with. For example, when using smaller versions of the medical device 110 that are compatible with smaller guidewires, it may be desirable to add such structural support.

The support member 140 extends along the distal tip region 120. This may include the support member 140 extending distally to a position at or adjacent to the distal end of the distal tip region 120 and/or extending proximally to a position at or adjacent to the proximal end of the distal tip region 120. In some instances, the support member 140 may have a tapered distal end. Other portions of the support member 140 may include tapers, steps in diameter, and/or the like, which may or may not be intermixed with constant diameter regions. The support member 140 extends proximally from the distal tip region 120 and along at least a portion of the remainder of the shaft 112. This may include the support member 140 extending along substantially the full length of the shaft 112 (or along only a portion of the length of the shaft 112).

It can be seen in FIG. 5 that the distal end region 116 of the shaft 112 and the distal tip region 120 may overlap and/or otherwise include an overlapping region where the distal end region 116 of the shaft 112 is bonded with or coupled to the distal tip region 120 (e.g., via a reflow, extrusion, adhesive bond, thermal bond, etc.). This overlap may be understood to have a length corresponding to the length of section/region where the distal end region 116 and the distal tip region 120 overlap. In some instances, the length of this overlapping section may be on the order of about 0.5-5cm, or about 0.5-2cm, or about 1cm. However, in other embodiments, the length may differ. For example, the length may be on the order of about 1-30cm, or about 5-25cm, or about 10-20cm. In some instances, this may increase the overall length of the guidewire lumen 134. For example, the length of the "non-overlapping" portion of the distal tip region 120 may be kept the same while the overall length of the distal tip region 120 may be increased so as to accommodate the increased length of the portion of the distal region 120 overlapping with the distal end region 116 of the shaft 112. In other instances, the "non-overlapping" portion of the distal tip region 120 may be shorter, for example due to more of the length of the distal tip region 120 overlapping with the distal end region 116 of the shaft 112.

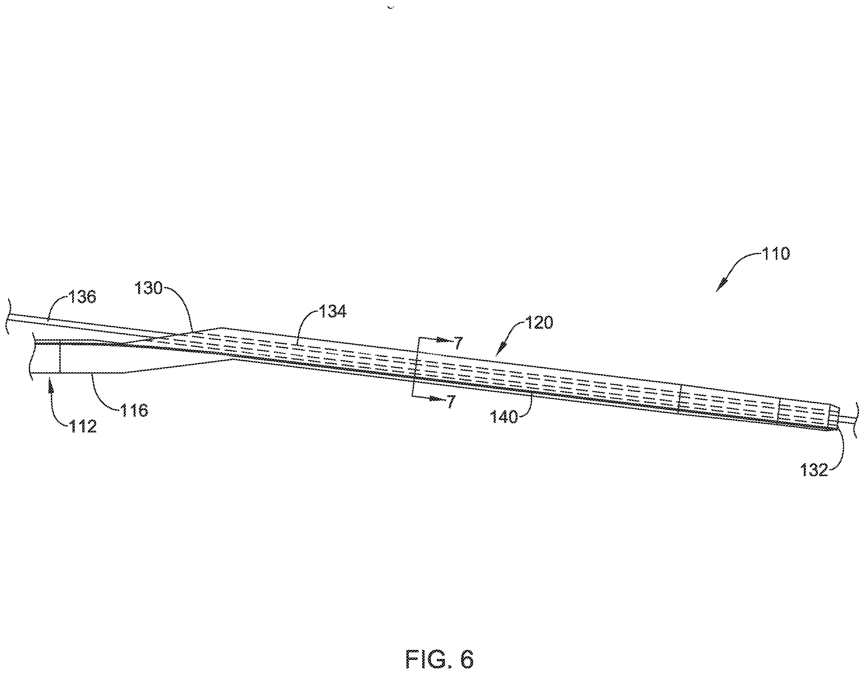

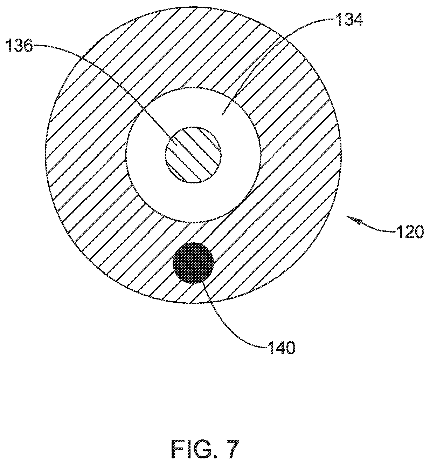

FIG. 6 illustrates the medical device 110 with a guidewire 136 passing through the lumen 134. and a cross-sectional view taken through line 7-7 in FIG. 6 is shown in FIG. 7. Here it can be seen that the support member 140 is disposed in the wall of the tubular structure forming the distal tip region 120. Likewise, portions of the support member 140 extending proximally from the distal tip region 120 may also be disposed within the wall of the shaft 112.

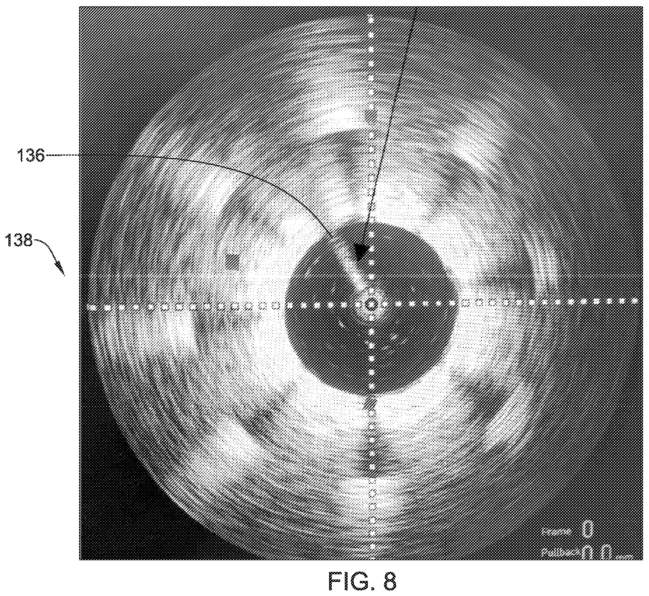

The arrangement of the support member 140 is particularly designed in a manner that helps to reduce the likelihood of the support member 140 being visualized during an imaging procedure. As can be seen, the support member 140 may be radially and/or circumferentially aligned with the guidewire 136 passing through the lumen 134. More proximal portions of the support member 140, for example those portions extending along the shaft 112 such as those portion that may overlap locations where the imaging assembly may be used to generate images, are radially and/or circumferentially aligned with the guidewire 136 (i.e., radially and/or circumferentially aligned with where the guidewire 136 will extend along the exterior of the shaft 112). Because of this alignment, the guidewire 136 may mask or otherwise overlie the support member such that a two-dimensional IVUS image 138 (e.g., generated by the imaging assembly of the medical device 110) will only show the guidewire 136 (e.g., not a separate marking corresponding to the support member 140) as depicted in FIG. 8. Thus, the alignment of the support member 140 with the guidewire 136 helps to reduce an imaging markings or artifacts that might otherwise be visible if these structures are not aligned.

The materials that can be used for the various components of the medical device 10 (and/or other medical devices disclosed herein) and the various tubular members disclosed herein may include those commonly associated with medical devices. For simplicity purposes, the following discussion makes reference to the medical device 10. However, this is not intended to limit the devices and methods described herein, as the discussion may be applied to other medical devices (e.g., including the medical device 110) and/or components thereof.

The medical device 10 and/or other components thereof may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene, low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

In at least some embodiments, portions or all of the medical device 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the medical device 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of medical device 10 to achieve the same result.

In some embodiments, a degree of Magnetic Resonance Imaging (MRI) compatibility is imparted into the medical device 10. For example, medical device 10, or portions thereof, may be made of a material that does not substantially distort the image and create substantial artifacts (e.g., gaps in the image). Certain ferromagnetic materials, for example, may not be suitable because they may create artifacts in an MRI image. The medical device 10, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. In any case, the scope of the invention is defined by the appended claims.

## Claims

1. An intravascular imaging catheter (10) comprising:
an elongate sheath (12, 112) having a distal tip region (20, 120) and a body region (16, 116);
wherein the distal tip region includes a first port (30, 130), a second port (32, 132), and a guidewire lumen (34, 134) extending between the first port and the second port;
a core wire (140) disposed within the elongate sheath along the distal tip region and extending proximally along at least a portion of the body region; and
an imaging core (22) disposed within the elongate sheath,
**characterised in that** the core wire is configured to be radially and/or circumferentially aligned with a guide wire (36, 136) extending along an exterior of the elongate sheath during use of the intravascular imaging catheter.

2. The intravascular imaging catheter of claim 1, wherein the core wire has a tapered distal end.

3. The intravascular imaging catheter of any one of claims 1-2, wherein the core wire extends to a distal end of the elongate sheath.

4. The intravascular imaging catheter of any one of claims 1-3, wherein the core wire extends proximally from the distal tip region of the elongate sheath.

5. The intravascular imaging catheter of any one of claims 1-4, wherein the core wire is aligned with the guidewire lumen.

6. The intravascular imaging catheter of any one of claims 1-5, wherein the core wire is radially aligned with the guidewire lumen.

7. The intravascular imaging catheter of any one of claims 1-5, wherein the core wire is circumferentially aligned with the guidewire lumen.

8. The intravascular imaging catheter of any one of claims 1-5, wherein the core wire is disposed within a wall of the elongate sheath.

9. The intravascular imaging catheter of any one of claims 1-8, wherein the core wire is configured to be aligned with a guidewire extending through the guidewire lumen during use of the intravascular imaging catheter.

10. The intravascular imaging catheter of any one of claims 1-9, wherein the imaging core includes an ultrasound transducer.

11. The intravascular imaging catheter of any one of claims 1-10, wherein the imaging core is slidably disposed within the elongate sheath.

12. An intravascular imaging catheter (10), comprising:
a sheath (12, 112) having a proximal end region (14), a body region (16, 116), and a distal tip region (20, 120);
wherein the distal tip region includes a first port (30, 130), a second port (32, 132), and a guidewire lumen (34, 134) extending between the first port and the second port;
a support member (140) disposed within a wall of the sheath along the distal tip region;
wherein the support member is aligned with the guidewire lumen; and
an imaging core (22) disposed within the sheath, the imaging core including an ultrasound transducer, and
wherein the support member extends along the body region **characterised in that** the support member is configured to be radially and/or circumferentially aligned with a guidewire (36, 136) extending along an exterior of the sheath during use of the intravascular imaging catheter.

13. The intravascular imaging catheter of claim 12, wherein the support member is configured to be aligned with a guidewire extending through the guidewire lumen during use of the intravascular imaging catheter.

14. A method for manufacturing an intravascular imaging catheter, the catheter comprising an elongate sheath having a distal tip region and an imaging core disposed within the elongate sheath, the method comprising:
disposing a core wire within a wall of the distal tip region of the elongate sheath, the distal tip region having a first port, a second port, and a guidewire lumen extending between the first port and the second port;
wherein the elongate sheath includes a body region;
wherein disposing the core wire within a wall of the distal tip region of the elongate sheath further comprises extending the core wire proximally along at least a portion of the body region of the elongate sheath;
wherein the core wire is aligned with the guidewire lumen; and
wherein the core wire is configured to be radially and/or circumferentially aligned with a guidewire extending along an exterior of the elongate sheath during use of the intravascular imaging catheter.

## Patentansprüche

1. Intravaskulärer Bildgebungskatheter (10), aufweisend:
einen langgestreckten Schaft (12, 112) mit einem distalen Spitzenbereich (20, 120) und einem Körperbereich (16, 116);
wobei der distale Spitzenbereich eine erste Öffnung (30, 130), eine zweite Öffnung (32, 132) und ein sich zwischen der ersten und der zweiten Öffnung erstreckendes Führungsdrahtlumen (34, 134) beinhaltet;
einen Kerndraht (140), der in dem langgestreckten Schaft entlang des distalen Spitzenbereichs angeordnet ist und sich entlang mindestens eines Abschnitts des Körperbereichs proximal erstreckt; und
einen Bildgebungskern (22), der in dem langgestreckten Schaft angeordnet ist, **dadurch gekennzeichnet, dass** der Kerndraht dazu konfiguriert ist, radial und/oder oder in Umfangsrichtung mit einem sich während des Gebrauchs des intravaskulären Bildgebungskatheters entlang eines Äußeren des langgestreckten Schafts erstreckenden Führungsdraht (36, 136) ausgerichtet zu sein.

2. Intravaskulärer Bildgebungskatheter nach Anspruch 1, wobei der Kerndraht ein verjüngtes distales Ende aufweist.

3. Intravaskulärer Bildgebungskatheter nach einem der Ansprüche 1-2, wobei sich der Kerndraht zu einem distalen Ende des langgestreckten Schafts erstreckt.

4. Intravaskulärer Bildgebungskatheter nach einem der Ansprüche 1-3, wobei sich der Kerndraht von dem distalen Spitzenbereich des langgestreckten Schafts proximal erstreckt.

5. Intravaskulärer Bildgebungskatheter nach einem der Ansprüche 1-4, wobei der Kerndraht mit dem Führungsdrahtlumen ausgerichtet ist.

6. Intravaskulärer Bildgebungskatheter nach einem der Ansprüche 1-5, wobei der Kerndraht radial mit dem Führungsdrahtlumen ausgerichtet ist.

7. Intravaskulärer Bildgebungskatheter nach einem der Ansprüche 1-5, wobei der Kerndraht in Umfangsrichtung mit dem Führungsdrahtlumen ausgerichtet ist.

8. Intravaskulärer Bildgebungskatheter nach einem der Ansprüche 1-5, wobei der Kerndraht in einer Wand des langgestreckten Schafts angeordnet ist.

9. Intravaskulärer Bildgebungskatheter nach einem der Ansprüche 1-8, wobei der Kerndraht dazu konfiguriert ist, mit einem sich während des Gebrauchs des intravaskulären Bildgebungskatheters durch das Führungsdrahtlumen erstreckenden Führungsdraht ausgerichtet zu sein.

10. Intravaskulärer Bildgebungskatheter nach einem der Ansprüche 1-9, wobei der Bildgebungskern einen Ultraschallwandler beinhaltet.

11. Intravaskulärer Bildgebungskatheter nach einem der Ansprüche 1-10, wobei der Bildgebungskern in dem langgestreckten Schaft verschiebbar angeordnet ist.

12. Intravaskulärer Bildgebungskatheter (10), aufweisend:
einen Schaft (12, 112) mit einem proximalen Endbereich (14), einem Körperbereich (16, 116) und einem distalen Spitzenbereich (20, 120);
wobei der distale Spitzenbereich eine erste Öffnung (30, 130), eine zweite Öffnung (32, 132) und ein sich zwischen der ersten Öffnung und der zweiten Öffnung erstreckendes Führungsdrahtlumen (34, 134) beinhaltet;
ein Stützelement (140), das in einer Wand des Schafts entlang des distalen Spitzenbereichs angeordnet ist;
wobei das Stützelement mit dem Führungsdrahtlumen ausgerichtet ist; und
einen Bildgebungskern (22), der in dem Schaft angeordnet ist, wobei der Bildgebungskern einen Ultraschallwandler beinhaltet, und
wobei sich das Stützelement entlang des Körperbereichs erstreckt, **dadurch gekennzeichnet, dass** das Stützelement dazu konfiguriert ist, radial und/oder in Umfangsrichtung mit einem sich während des Gebrauchs des intravaskulären Bildgebungskatheters entlang eines Äußeren des Schafts erstreckenden Führungsdraht (36, 136) ausgerichtet zu sein.

13. Intravaskulärer Bildgebungskatheter nach Anspruch 12, wobei das Stützelement dazu konfiguriert ist, mit einem sich während des Gebrauchs des intravaskulären Bildgebungskatheters durch das Führungsdrahtlumen erstreckenden Führungsdraht ausgerichtet zu sein.

14. Verfahren zum Herstellen eines intravaskulären Bildgebungskatheters, wobei der Katheter einen langgestreckten Schaft mit einem distalen Spitzenbereich und einen in dem langgestreckten Schaft angeordneten Bildgebungskern aufweist, wobei das Verfahren aufweist:
Anordnen eines Kerndrahts in einer Wand des distalen Spitzenbereichs des langgestreckten Schafts, wobei der distale Spitzenbereich eine erste Öffnung, eine zweite Öffnung und ein sich zwischen der ersten Öffnung und der zweiten Öffnung erstreckenden Führungsdrahtlumen aufweist;
wobei der langgestreckte Schaft einen Körperbereich beinhaltet;
wobei das Anordnen des Kerndrahts in einer Wand des distalen Spitzenbereichs des langgestreckten Schafts ferner das proximale Verlegen des Kerndrahts entlang mindestens eines Abschnitts des Körperbereichs des langgestreckten Schafts aufweist;
wobei der Kerndraht mit dem Führungsdrahtlumen ausgerichtet wird; und
wobei der Kerndraht dazu konfiguriert ist, radial und/oder in Umfangsrichtung mit einem sich während des Gebrauchs des intravaskulären Bildgebungskatheters entlang eines Äußeren des langgestreckten Schafts erstreckenden Führungsdraht ausgerichtet zu sein.

## Revendications

1. Cathéter d'imagerie intravasculaire (10) comprenant :
une gaine allongée (12, 112) ayant une région de pointe distale (20,120) et une région de corps (16, 116) ;
dans lequel la région de pointe distale comprend un premier orifice (30, 130), un deuxième orifice (32, 132) et une lumière de fil-guide (34, 134) s'étendant entre le premier orifice et le deuxième orifice ;
un fil central (140) disposé à l'intérieur de la gaine allongée le long de la région de pointe distale et s'étendant, de manière proximale, le long d'au moins une partie de la région de corps ; et
un noyau d'imagerie (22) disposé à l'intérieur de la gaine allongée,
**caractérisé en ce que** le fil central est configuré pour être aligné de manière radiale et/ou circonférentielle avec un fil-guide (36, 136) s'étendant le long d'un extérieur de la gaine allongée pendant l'utilisation du cathéter d'imagerie intravasculaire.

2. Cathéter d'imagerie intravasculaire selon la revendication 1, dans lequel le fil central a une extrémité distale progressivement rétrécie.

3. Cathéter d'imagerie intravasculaire selon l'une quelconque des revendications 1 à 2, dans lequel le fil central s'étend vers une extrémité distale de la gaine allongée.

4. Cathéter d'imagerie intravasculaire selon l'une quelconque des revendications 1 à 3, dans lequel le fil central s'étend de manière proximale à partir de la région de pointe distale de la gaine allongée.

5. Cathéter d'imagerie intravasculaire selon l'une quelconque des revendications 1 à 4, dans lequel le fil central est aligné avec la lumière de fil-guide.

6. Cathéter d'imagerie intravasculaire selon l'une quelconque des revendications 1 à 5, dans lequel le fil central est aligné, de manière radiale, avec la lumière de fil-guide.

7. Cathéter d'imagerie intravasculaire selon l'une quelconque des revendications 1 à 5, dans lequel le fil central est aligné, de manière circonférentielle, avec la lumière de fil-guide.

8. Cathéter d'imagerie intravasculaire selon l'une quelconque des revendications 1 à 5, dans lequel le fil central est disposé à l'intérieur d'une paroi de la gaine allongée.

9. Cathéter d'imagerie intravasculaire selon l'une quelconque des revendications 1 à 8, dans lequel le fil central est configuré pour être aligné avec un fil-guide s'étendant à travers la lumière de fil-guide pendant l'utilisation du cathéter d'imagerie intravasculaire.

10. Cathéter d'imagerie intravasculaire selon l'une quelconque des revendications 1 à 9, dans lequel le noyau d'imagerie comprend un transducteur à ultrasons.

11. Cathéter d'imagerie intravasculaire selon l'une quelconque des revendications 1 à 10, dans lequel le noyau d'imagerie est disposé, de manière coulissante, à l'intérieur de la gaine allongée.

12. Cathéter d'imagerie intravasculaire (10) comprenant :
une gaine (12, 112) ayant une région d'extrémité proximale (14), une région de corps (16, 116) et une région de pointe distale (20, 120) ;
dans lequel la région de pointe distale comprend un premier orifice (30, 130), un deuxième orifice (32, 132), et une lumière de fil-guide (34, 134) s'étendant entre le premier orifice et le deuxième orifice ;
un élément de support (140) disposé à l'intérieur d'une paroi de la gaine le long de la région de pointe distale ;
dans lequel l'élément de support est aligné avec la lumière de fil-guide ; et
un noyau d'imagerie (22) disposé à l'intérieur de la gaine, le noyau d'imagerie comprenant un transducteur à ultrasons, et
dans lequel l'élément de support s'étend le long de la région de corps,
**caractérisé en ce que** l'élément de support est configuré pour être aligné, de manière radiale et/ou circonférentielle, avec un fil-guide (36, 136) s'étendant le long d'un extérieur de la gaine pendant l'utilisation du cathéter d'imagerie intravasculaire.

13. Cathéter d'imagerie intravasculaire selon la revendication 12, dans lequel l'élément de support est configuré pour être aligné avec un fil-guide s'étendant à travers la lumière de fil-guide pendant l'utilisation du cathéter d'imagerie intravasculaire.

14. Procédé pour fabriquer un cathéter d'imagerie intravasculaire, le cathéter comprenant une gaine allongée ayant une région de pointe distale et un noyau d'imagerie disposé à l'intérieur de la gaine allongée, le procédé comprenant le fait de :
disposer un fil central à l'intérieur d'une paroi de la région de pointe distale de la gaine allongée,
la région de pointe distale ayant un premier orifice, un deuxième orifice et une lumière de fil-guide s'étendant entre le premier orifice et le deuxième orifice ;
dans lequel la gaine allongée comprend une région de corps ;
dans lequel le fait de disposer le fil central à l'intérieur d'une paroi de la région de pointe distale de la gaine allongée comprend en outre le fait d'étendre le fil central de manière proximale le long d'au moins une partie de la région de corps de la gaine allongée ;
dans lequel le fil central est aligné avec la lumière de fil-guide ; et
dans lequel le fil central est configuré pour être aligné, de manière radiale et/ou circonférentielle, avec un fil-guide s'étendant le long d'un extérieur de la gaine allongée pendant l'utilisation du cathéter d'imagerie intravasculaire.
